# EUROPÄISCHE PATENTSCHRIFT

(11) **EP 1 399 173 B1**
(45) Veröffentlichungstag und Bekanntmachung des Hinweises auf die Patenterteilung: **14.11.2007**
(21) Anmeldenummer: 02753064.1
(22) Anmeldetag: 29.05.2002
(51) Int. Cl.: A61P 37/00, A61K 36/23, A61K 36/29, A61K 36/86

(54) **PHARMAZEUTISCHE ZUBEREITUNG ENTHALTEND WIRKSTOFFE AUS DEN PFLANZEN CENTELLA ASIATICA, MAHONIA AQUIFOLIUM UND VIOLA TRICOLOR**
PHARMACEUTICAL PREPARATION CONTAINING ACTIVE INGREDIENTS EXTRACTED FROM THE PLANTS CENTELLA ASIATICA, MAHONIA AQUIFOLIUM AND VIOLA TRICOLOR
PREPARATION PHARMACEUTIQUE CONTENANT DES INGREDIENTS ACTIFS EXTRAITS DE CENTELLA ASIATICA, MAHONIA AQUIFOLIUM ET VIOLA TRICOLOR

(30) Priorität: 30.05.2001 DE 20109044 U
(43) Veröffentlichungstag der Anmeldung: 24.03.2004
(73) Patentinhaber: Weber & Weber GmbH & CO. KG, 82266 Inning (DE)
(72) Erfinder: RITTINGHAUSEN, Reiner, 82266 Inning/Bachern (DE); KOCH, Volkmar, 86919 Utting/Ammersee (DE)
(74) Vertreter: Michalski, Stefan
(86) Internationale Anmeldenummer: PCT/EP2002/005920
(87) Internationale Veröffentlichungsnummer: WO 2002/096447

(56) Entgegenhaltungen:
- EP-A- 0 867 447
- WO-A-01/19365
- WO-A-94/02118
- JP-A- 4 086 632
- RO-A- 103 683
- K. MÜLLER ET AL.: "The antipsoriatic Mahonia aquifolium and its active constituents; II. Antiproliferative activity against cell growth of human keratinocytes" PLANTA MEDICA, Bd. 61, Nr. 1, Februar 1995 (1995-02), Seiten 74-75, XP000099609
- PATENT ABSTRACTS OF JAPAN vol. 1999, no. 01, 29. Januar 1999 (1999-01-29) & JP 10 279491 A (KAO CORP), 20. Oktober 1998 (1998-10-20)

## Beschreibung

Die vorliegende Erfindung betrifft eine pharmazeutische Zubereitung und deren Verwendung zur Behandlung von allergischen Hauterkrankungen, atopisches Ekzem, Hauterkrankungen durch Pilze, Urtikaria, Neurodermitis, Psoriasis, Schuppenflechte, Hautekzemen, Hautjucken, entzündlichen Hauterkrankungen und/oder Viruserkrankungen, umfassend Windpocken, Varicellen, Herpes und Gürtelrose.

Pflanzenextrakte aus Centella asiatica, Mahonia aquifolium, Viola tricolor und/oder aus deren Pflanzenteilen zur Behandlung von Viruserkrankungen, insbesondere von Virus verursachten Hauterkrankungen oder von Virus verursachten Hautververänderungen, umfassend Windpocken, Varicellen, Herpes, Röteln, und Gürtelrose, sind aus dem Stand der Technik nicht bekannt.

Hautekzeme sind gerötete und schuppende, manchmal auch nässende Hautveränderungen. Typische Stellen, an denen sie auftreten, sind Ellenbogen, Kniekehlen, Nacken, Hals und Gesicht. Charakteristisch für Patienten mit Hautjuckreiz ist ihre trockene Haut.

Der Juckreiz ist ein Alarmsignal der Haut auf eine geänderte äußere oder innere Situation. Die Juckreizempfindung wird durch verschiedene mechanische, physikalische und chemische Einflüsse verursacht. Auch psychische Faktoren spielen eine Rolle.

Juckreiz wird hauptsächlich durch vier Mechanismen ausgelöst, die teilweise ineinander greifen oder sich gegenseitig beeinflussen können:
1. Freisetzung von Juckreiz auslösenden Gewebestoffen, beispielsweise Histamin, aus den Abwehrzellen der Haut, beispielsweise aus den Mastzellen;
2. Freisetzung von Juckreiz auslösenden und Entzündungen verursachenden proteolytischen Enzymen, beispielsweise aus den Phagozyten;
3. direkte Wirkung auf nervale Rezeptoren und damit Ausschüttung von Nervenbotenstoffen, u.a. Neuropeptide;
4. Juckreizstimulation im Gehirn.

Ein derartiger Reiz in oder auf der Haut löst einen sogenannten Kratzreflex aus. Da eine Entzündung der Haut über die Erregung von Nervenendigungen erneuten Juckreiz auslösen kann, kann es zu einem Teufelskreis aus Kratzen, erneutem Juckreiz, noch stärkerem Kratzen usw. kommen.

Neurodermitis ist einer der häufigsten Hauterkrankungen, die einen Juckreiz bzw. Kratzen auslösen. Ihr Krankheitsbild ist von einem wechselnden, sehr unterschiedlich aussehenden Ekzem geprägt.

Ein Präparat zur Behandlung von juckenden Ekzemen wird kommerziell angeboten und vertrieben. Es enthält als Wirkstoffe 1 Gew.-% Perubalsam (Balsamum peruvianum) und 5 Gew.-% Zinkoxid. Weitere Bestandteile sind Extrakte aus Centella asiatica, Mahonia aquifolium, Viola tricolor, Acidum arsenicosum, Calendula officinalis, Lytta vesicatoria und Semecarpus anacardium. Vorgenanntes Präparat weist jedoch Nachteile auf.

Perubalsam ist ein seit langem bekannter Wirkstoff, welcher beispielsweise in Arzneimitteln hauptsächlich äußerlich als Wundheilmittel sowie als Bestandteil von Hämorrhoidenmitteln, Antiscabiosum (gegen Krätze), sowie bei Einreibungen Anwendung findet. Perubalsam bzw. Balsamum peruvianum umfasst mindestens 45% und maximal 75% mit Ether extrahierbare Zimtsäure- und Benzoesäurebenzylester, ferner 25-30% Harze, Benzoesäure, Zimtsäure, Vanillin, Nerolidol und Farnesol. Neben seiner antiseptischen Wirkung führt Perubalsam jedoch relativ häufig zu unerwünschten Nebenwirkungen in Form von Kontaktallergien.

Ein weiterer Nachteil des vorstehend genannten Präparats zur Behandlung von juckenden Ekzemen besteht in dem hohen Gehalt an Zinkoxid, das als Bestandteil von Salben bzw. Pasten zu einer Austrocknung der Haut und damit zu einem unangenehmen Hautgefühl führt.

Es wäre sehr wünschenswert, eine pharmazeutische Zubereitung zur Behandlung von juckenden Ekzemen wie beispielsweise Neurodermitis zur Verfügung zu haben, welche die oben geschilderten Nachteile vermeidet. Insbesondere wäre es wünschenswert, eine pharmazeutische Zubereitung zur Verfügung zu haben, welche zu einem angenehmen Hautgefühl für den Patienten führt und keinerlei Nebenwirkungen in Form von allergischen Hautreaktionen aufweist.

Außerdem wäre es sehr wünschenswert, eine pharmazeutische Zubereitung zur Verfügung zu stellen, die zusätzlich zur Behandlung von Viruserkrankungen, insbesondere zur Behandlung von Virus verursachten Hauterkrankungen und/oder Virus verursachten Hautveränderungen, umfassend Windpocken, Varicellen, Herpes, Röteln und Gürtelrose, geeignet ist.

Aufgabe der vorliegenden Erfindung ist es daher, ein pharmazeutisches Präparat zur Verfügung zu stellen, welches die Nachteile des Standes der Technik überwindet und zur Behandlung von Hautekzemen, juckenden Ekzemen, insbesondere Hautjucken, wie beispielsweise bei der Neurodermitis, eingesetzt werden kann.

Eine weitere Aufgabe der vorliegenden Erfindung ist es, ein pharmazeutisches Präparat geeignet zur Behandlung von Viruserkrankungen, insbesondere zur Behandlung von Virus verursachten Hauterkrankungen und/oder Virus verursachten Hautveränderungen, umfassend Windpocken, Varicellen, Herpes, Röteln und Gürtelrose, zur Verfügung zu stellen.

Noch eine Aufgabe der vorliegenden Erfindung ist es eine pharmazeutische Zubereitung zur Verfügung zu stellen, die zur Behandlung von allergischen Hauterkrankungen, atopisches Ekzem, Hauterkrankungen durch Pilze, Urtikaria, Neurodermitis, Psoriasis, Schuppenflechte und/oder entzündlichen Hauterkrankungen, geeignet ist.

Weitere Aufgaben ergeben sich aus den nachfolgenden Beschreibung der Erfindung.

Erfindungsgemäß wird zur Behandlung von allergischen Hauterkrankungen, atopisches Ekzem, Hauterkrankungen durch Pilze, Urtikaria, Neurodermitis, Psoriasis, Schuppenflechte, Hautekzemen, Hautjucken, entzündlichen Hauterkrankungen und/oder Viruserkrankungen, umfassend Windpocken, Varicellen, Herpes und Gürtelrose die Verwendung einer pharmazeutischen Zubereitung zur Herstellung eines Arzneimittels vorgeschlagen, wobei die pharmazeutische Zubereitung natürliche und/oder synthetische Wirkstoff(e) aus den Pflanzen Centella asiatica, Mahonia aquifolium, Viola tricolor und/oder aus deren Pflanzenteilen umfasst. Ferner wird erfindungsgemäß eine pharmazeutische Zubereitung bereitgestellt, die natürliche und/oder synthetische Wirkstoff(e) aus den Pflanzen Centella asiatica, Mahonia aquifolium, Viola tricolor und/oder aus deren Pflanzenteilen umfasst, wobei die Zubereitung einen Gehalt von weniger als 5 Gew.-% Zinkoxid und/oder einen Gehalt von weniger als 1 Gew.-% Perubalsam aufweist.

Vorteilhaft ist die erfindungsgemäße Verwendung der pharmazeutischen Zubereitung zur Behandlung von Viruserkrankungen, insbesondere zur Behandlung von Virus verursachten Hauterkrankungen und/oder Virus verursachten Hautveränderungen.

Vorteilhafte Verwendungen und Ausgestaltungen der erfindungsgemäßen pharmazeutischen Zubereitung sind in den Unteransprüchen definiert.

Alle Angaben in Gew.-% beziehen sich, wenn nicht anders angegeben, auf die Gesamtzusammensetzung der pharmazeutischen Zubereitung.

Die Wirkstoffe der erfindungsgemäßen Zubereitung werden vorzugsweise als Extrakt aus den Pflanzen Centella asiatica, Mahonia aquifolium, Viola tricolor und/oder aus deren Pflanzenteilen und gegebenenfalls aus weiteren nachstehend beschriebenen Pflanzen bzw. Pflanzenteilen oder Tieren gewonnen. Besonders bevorzugt wird eine pflanzliche Urtinktur hergestellt, die natürliche und/oder synthetische Wirkstoff(e) aus den Pflanzen Centella asiatica, Mahonia aquifolium, Viola tricolor und/oder aus deren Pflanzenteilen und gegebenenfalls aus weiteren nachstehend beschriebenen Pflanzen bzw. Pflanzenteilen umfasst.

Unter Extrakten werden im Rahmen der vorliegenden Erfindung konzentrierte, gegebenenfalls auf einen bestimmten Wirkstoffgehalt eingestellte Zubereitungen aus Wirkstoffen verstanden.

Alkoholische Extrakte können direkt als Flüssigextrakte verwendet werden oder nach Entfernen des Auszugsmittels als Trockenextrakte.

Üblicherweise können alkoholische Extrakte, beispielsweise mit einem Ethanolgehalt von ≥ 30 Gew.-% und einem Wassergehalt von ≤ 70 Gew.-%, bezogen auf das Gesamtgewicht des Extraktionsmittel, verwendet werden. Vorzugsweise weisen die alkoholischen Extrakte ≥ 42 Gew.-%, ≥ 62 Gew.-%, ≥ 86 Gew.-% oder ≥ 94 Gew.-% Ethanol auf, wobei der Rest Wasser ist.

Für die Extraktion aus den drei Pflanzen, auch als Drogen bezeichnet, Centella asiatica, Mahonia aquifolium, Viola tricolor und/oder aus deren Pflanzenteilen werden als Extraktionsmittel bevorzugt die vorstehenden Ethanol/Wasser Gemische verwendet.

Centella asiatica, kann man beispielsweise nach Vorschrift 4a HAB (Homöopathisches Arzneibuch 2001) mit 62 Gew.-% Ethanol und 38 Gew.-% Wasser, extrahieren. Für die Extraktion verwendet man bevorzugt die getrockneten oberirdischen Teile von Centella asiatica (Linne) Urban.

Mahonia aquifolium, kann man beispielsweise nach Vorschrift 4a HAB (Homöopathisches Arzneibuch 2001) mit 62 Gew.-% Ethanol und 38 Gew.-% Wasser. Für die Extraktion verwendet man bevorzugt die Ast- und Zweigrinde und die getrockneten Zweigspitzen von Mahonia aquifolium (Pursh).

Viola tricolor, kann man beispielsweise nach Vorschrift 2a HAB (Homöopathisches Arzneibuch 2001) mit 86% Gew.-% Ethanol und 14 Gew.-% Wasser. Verwendet werden vorzugsweise die frischen oberirdischen Teile von Viola tricolor.

Es können für die Extraktion aus diesen 3 Drogen, Centella asiatica, Mahonia aquifolium, Viola tricolor, auch andere Ethanol/Wasser Gemische verwendet werden.

Lipophilere Auszüge lassen sich mit entsprechendem Auszugsmitteln C₂-C₈-Alkylverbindungen, wie Pentan, n-Hexan, oder mittels verdichtetem Ethylen, Ethan oder Kohlendioxid unter überkritischen Bedingungen herstellen.

In der WO 00/12107 werden Verfahren zur Extraktion von Pflanzen oder Pflanzenteilen mit Kohlendioxid, Ethylen sowie Ethan unter überkritischen Bedingungen beschrieben, auf die hier im vollem Umfang Bezug genommen wird. Die Extraktion mit unterkritischem sowie überkritischem Kohlendioxid ist dem Fachmann allgemein bekannt.

Außerdem werden Verfahren zur Herstellung von Pflanzen- bzw. Drogenextrakten in Hagers Handbuch Band 2, Springerverlag 1991, Seiten 1024 - 1031, beschrieben, auf die hier ebenfalls im vollem Umfang Bezug genommen werden.

Nach Entfernen des Auszugsmittels bzw. Extraktionsmittel erhält man Trocken- oder Spissumextrakte. Trockenextrakte lassen sich zu Pulver verarbeiten. Bei Spissumextrakten handelt es sich um zähflüssige Extrakte, häufig mit einem Wassergehalt von ≤ 5 Gew.-% Wasser, bezogen auf den Spissumextrakt.

Unter einer Urtinktur werden im Rahmen der vorliegenden Erfindung Mischungen pflanzlicher Preßsäfte und/oder pflanzlicher Auszüge mit Lösungsmitteln wie Ethanol, Wasser und/oder Glycerol, bzw. Auszüge von Pflanzen oder Tieren, deren Absonderungen oder deren Teilen verstanden. Urtinkturen werden vorzugsweise mit flüssigen Arzneimittelträgern wie Ethanol verschiedener Konzentration, Wasser und/oder Glycerol bereitet.

Unter synthetischen Wirkstoffen werden im Rahmen der vorliegenden Erfindung Naturstoffidentische bzw. Naturstoff-ähnliche Wirkstoffe verstanden, die totalsynthetisch bzw. teilweise synthetisch hergestellt worden sind.

Es wurde überraschenderweise gefunden, dass eine erfindungsgemäße pharmazeutische Zubereitung, die weniger als 1 Gew.-% Perubalsam umfasst, keinerlei Nebenwirkungen, beispielsweise in Form von allergischen Hautreaktionen, hervorruft. Gleichzeitig bleibt jedoch die herausragende juckreizlindernde Wirkung der pharmazeutischen Zubereitung gewährleistet. So wird vermieden, dass durch den Juckreiz ein Kratzreflex ausgelöst wird, der zu einem erneuten verstärkten Juckreiz führt. Somit umfasst die Zubereitung bei einer Ausführungsform der vorliegenden Erfindung bevorzugt ≤ 0,5 Gew.-% , weiter bevorzugt ≤ 0,1 Gew.-% und besonders bevorzugt ≤ 0,01 Gew.-% Perubalsam.

In einer bevorzugten Ausführungsform umfasst die pharmazeutische Zubereitung kein Perubalsam. Dadurch werden allergische Hautreaktionen z.B. bei wiederholter Behandlung oder gar Langzeittherapie von Juckreizen und Hautekzemen insbesondere auch bei sehr empfindlichen Patienten vermieden.

Überraschend ist auch die Wirksamkeit der pharmazeutischen Zubereitung gegen Pilze und Viren, insbesondere gegen von Viren und/oder Pilzen verursachte Hauterkrankungen und/oder Hautveränderungen.

In einer weiteren bevorzugten Ausführungsform der vorliegenden Erfindung umfasst die pharmazeutische Zubereitung ferner bevorzugt ≤ 3 Gew.-%, weiter bevorzugt ≤ 1 Gew.-%, besonders bevorzugt ≤ 0,1 Gew.-% und am meisten bevorzugt kein Zinkoxid. Bevorzugte Untergrenzen für den Zinkoxid-Gehalt in der erfindungsgemäßen pharmazeutischen Zubereitung sind 0,5 Gew.-% und besonders bevorzugt 0,1 Gew.-% Zinkoxid. Am meisten bevorzugt enthält die erfindungsgemäße pharmazeutische Zubereitung kein Zinkoxid. Durch den geringen Zinkoxid-Gehalt der erfindungsgemäßen pharmazeutischen Zubereitung wird ein Austrocknen der Haut vermieden und somit ein angenehmes Hautgefühl durch die Behandlung mit der erfindungsgemäßen Zubereitung vermittelt.

Vorzugsweise umfaßt die erfindungsgemäße Zubereitung Wirkstoff(e), die aus den Pflanzen Centella asiatica, Mahonia aquifolium, Viola tricolor und/oder aus deren Pflanzenteilen und gegebenenfalls aus weiteren nachstehend beschriebenen Pflanzen bzw. Pflanzenteilen mittels Extraktion erhalten worden sind.

Die erfindungsgemäße Zubereitung kann auch als Urtinktur(en) vorliegen, die aus den Pflanzen Centella asiatica, Mahonia aquifolium, Viola tricolor und/oder aus deren Pflanzenteilen und gegebenenfalls aus weiteren nachstehend beschriebenen Pflanzen bzw. Pflanzenteilen erhalten worden sind.

Als einen der Bestandteile umfaßt die pharmazeutische Zubereitung bei einer bevorzugten Ausführungsform der vorliegenden Erfindung einen Extrakt, insbesondere eine Urtinktur, aus der Pflanze Centella asiatica und/oder aus deren Pflanzenteilen mit einem Gehalt von 1 Gew.-% bis 10 Gew.-%, vorzugsweise von 3 Gew.-% bis 7 Gew.-%, besonders bevorzugt von 4 Gew.-% bis 6 Gew.-% und am meisten bevorzugt von 5 Gew.-%.

Centella asiatica (asiatisches Wassernabelkraut) enthält etwa 0,1 Gew.-% ätherische Öle, Flavonolderivate, Ursanderivate, beispielsweise (freie) Asiatsäure und deren Trisaccharid-Ester. Wirkstoffe aus Centella asiatica sind effizient bei Hauterkrankungen mit Verdickungen und Juckreiz und kann in Form von Salben und Tinkturen zur Wundbehandlung, insbesondere zur Förderung der antimikrobiellen und antiphlogistischen Wirkung, eingesetzt werden.

Ferner umfasst die pharmazeutische Zubereitung gemäß der vorliegenden Erfindung bei einer bevorzugten Ausführungsform einen Extrakt, insbesondere eine Urtinktur, aus der Pflanze Mahonia aquifolium mit einem Gehalt von 1 Gew.-% bis 10 Gew.-%, vorzugsweise von 3 Gew.-% bis 7 Gew.-%, besonders bevorzugt von 4 Gew.-% bis 6 Gew.-% und am meisten bevorzugt von 5 Gew.-%.

Mahonia aquilfolium enthält als Inhaltsstoffe in der Stamm- und Wurzelrinde die Alkaloide Berberin, Oxyacanthin und Bermamin. Wirkstoffe aus Mahonia aquifolium (Berberis aquifolium) können vorteilhaft zur Behandlung von trockenen Hautausschlägen eingesetzt werden.

Ferner umfasst die erfindungsgemäße pharmazeutische Zubereitung bei einer bevorzugten Ausführungsform einen Extrakt, insbesondere eine Urtinktur, aus der Pflanze Viola tricolor mit einem Gehalt von 1 bis 10 Gew.-%, vorzugsweise 3 bis 7 Gew.-%, besonders bevorzugt 4 bis 6 Gew.-% und am meisten bevorzugt 5 Gew-%. Viola tricolor (Stiefmütterchen) enthält als Inhaltsstoffe Flavonoide, Spuren von ätherischen Ölen, das Glycosid Violutosid (Violutin), bei dem Methylsalicylat mit dem Disaccharid Vicianose verbunden ist, ferner Gerbstoff, Schleim und Zucker. Viola tricolor bzw. die darin enthaltenen Wirkstoffe sind wirksam bei der Behandlung von chronischen Hautleiden und Ausschlägen, besonders in der Kinderpraxis (Milchschorf), insbesondere auch bei der Behandlung von Ekzemen.

Vorzugsweise umfasst die erfindungsgemäße pharmazeutische Zubereitung ferner Farbstoffe. Beispiele für bevorzugte Farbstoffe sind Curcumin, Riboflavin und/oder β-Carotin.

Die erfindungsgemäße pharmazeutische Zubereitung kann als Salbe, Creme, Gel, Tablette oder Lösung vorliegen. Es ist allerdings erfindungsgemäß bevorzugt, dass die pharmazeutische Zubereitung als Salbe, insbesondere als hydrophile Salbe, besonders bevorzugt als wasserhaltige hydrophile Salbe vorliegt. Cremes sind im Rahmen der vorliegenden Erfindung ebenfalls mit Vorteil anzuwenden.

Die Salbengrundlage wird vorzugsweise mit der Maßgabe ausgewählt, dass wasserlösliche Pharmaka besser aus hydrophoben Salbengrundlagen und umgekehrt lipidlösliche Pharmaka rascher aus hydrophilen Salbengrundlagen in die Haut aufgenommen werden können. Als Salben- bzw. Cremegrundlage umfasst die erfindungsgemäße pharmazeutische Zubereitung somit vorzugsweise DAB- bzw. DAC-Grundlagen bzw. Bestandteile (DAB: Deutsches Arzneimittelbuch 2000/2001, DAC: Deutscher Arzneicodex 2000/2001) wie insbesondere Hydroxyethylcellulose und/oder Wollwachsalkoholsalbe.

Hydrophile Gel-Präparate sowie hydrophile Salben und hydrophile Cremes können Zinkoxid enthalten, da diese eine kühlende Wirkung besitzen und an die Haut Feuchtigkeit abgeben, so dass eine trockene Haut vermieden wird. Der Juckreiz wird ebenfalls durch die feuchtigkeitsspendende Eigenschaft der vorgenannten hydrophilen Formulierungen verringert.

Bei einer weiteren bevorzugten Ausführungsform der vorliegenden Erfindung umfasst die Zubereitung ferner natürliche und/oder synthetische Wirkstoff(e), insbesondere in Form einer Urtinktur, aus den Pflanzen Calendula officinalis und/oder Semecarpus anacardium und/oder aus deren Pflanzenteilen.

Calendula officinalis (Ringelblume) enthält als Inhaltsstoffe ätherische Öle, Calendulin, Calenduloside wie Saponine und Oleanolsäureglykoside, Triterpene, β-Carotin, Lycopin, Xanthophylle und Flavonoide. Die Wirkstoffe von Calendula officinalis weisen eine entzündungshemmende, bakterizide und granulationsfördernde Wirkung insbesondere in Salben bei schlechtheilenden Wunden und Geschwüren auf.

Semecarpus anacardium (Tintenbaum) enthält als Inhaltsstoffe phenolische Verbindungen wie Cardol, Gerbstoff, Harz und Farbstoff, die Samen enthalten etwa 47% fettes Öl mit Anacardsäure. Semecarpus anacardium bzw. dessen Wirkstoffe sind wirksam als Hautreizungsmittel und bei der Behandlung von Warzen und Hühneraugen.

Ferner kann die erfindungsgemäße Zubereitung als Wirkstoff Acidum arsenicosum (Arsen(III)oxid) umfassen.

Des weiteren kann die Zubereitung natürliche und/oder synthetische Wirkstoff(e) aus Lytta vesicatoria umfassen. Lytta vesicatoria ist ein Käfer, der etwa 0,5 bis 1 % Cantharidin, Harz, Fett und Farbstoff enthält. Zubereitungen von Lytta vesicatoria werden beispielsweise bei Verbrennungen verordnet.

Ferner kann die erfindungsgemäße pharmazeutische Zubereitung Wirkstoffe, ausgewählt aus der Gruppe bestehend aus Vitamin, insbesondere Vitamin E, β-Carotin, Biotin und Co-Enzym Q10 umfassen. Weitere vorteilhafte Wirkstoffe sind Curcumin und Curcuminwurzelstock-Extrakt.

Bei einer besonders bevorzugten Ausführungsform umfasst die Zubereitung Wirkstoffe, ausgewählt aus der Gruppe, bestehend aus ätherischen Ölen, Flavonolderivaten, Ursanderivaten, Berberin, Oxyacanthin, Bermamin, Violutosid (Violutin), Gerbstoffen, Zucker, Schleim, Calendulin, Calendulosiden, Triterpenen, β-Carotin, Lycopin, Xanthophyllen, Anacardsäure, Arsen(III)oxid, Cantharidin, Vitaminen, insbesondere Vitamin E, β-Carotin, Biotin, Co-Enzym Q10, Zinkoxid, mit Ether extrahierbaren Zimtsäure- und Benzoesäurebenzylestern, Harzen, Benzoesäure, Zimtsäure, Curcumin, Curcuminwurzelstock-Extrakt, Vanillin, Nerolidol und/oder Farnisol.

Des weiteren ist es bevorzugt, dass die erfindungsgemäße pharmazeutische Zubereitung gebrauchsfertig ist. Unter "gebrauchsfertig" im Sinne der vorliegenden Erfindung wird verstanden, dass die pharmazeutische Zubereitung als gebrauchsfertige Salbe vorliegt, die auf die juckenden bzw. entzündeten Hautstellen bzw. Hautekzeme aufgetragen werden kann.

Ein weiterer Gegenstand der vorliegenden Erfindung ist eine Tube, welche eine erfindungsgemäße pharmazeutische Zubereitung enthält.

Die vorliegende Erfindung betrifft ferner ein Verfahren zur Herstellung einer pharmazeutischen Zubereitung umfassend das Vermischen von Extrakten, insbesondere die Herstellung von Urtinkturen, aus Centella asiatica, Mahonia aquifolium, Viola tricolor, wobei ein Gehalt von 5 Gew.-% Zinkoxid und/oder ein Gehalt von 1 Gew.-% Perubalsam, bezogen auf die Gesamtzusammensetzung, ausgenommen wird.

Bei diesem erfindungsgemäßen Verfahren zur Herstellung einer pharmazeutischen Zubereitung zur Behandlung zur Herstellung eines Arzneimittels zur Behandlung von Neurodermitis, Psoriasis, Schuppenflechte, Windpocken, Varicellen, Herpes, Hautekzemen, Hautjucken und/oder entzündlichen Hauterkrankungen ist es bevorzugt, dass für die pharmazeutischen Zubereitung, insbesondere die Urtinkturen, aus Centella asiatica, Mahonia aquifolium und/oder Viola tricolor die vorstehend genannten Gewichtsbereiche beachtet werden.

Ferner ist es bevorzugt, dass bei dem erfindungsgemäßen Verfahren kein Perubalsam und/oder weniger als 5 Gew.-%, vorzugsweise weniger als 3 Gew.-%, besonders bevorzugt weniger als 1 Gew.-% und am meisten bevorzugt kein Zinkoxid zugegeben werden.

In einer weiteren bevorzugten Ausführungsform des erfindungsgemäßen Verfahrens wird die Zubereitung als Salbe bzw. Creme hergestellt, wobei als Salben- bzw. Cremegrundlage vorzugsweise DAB- bzw. DAC-Grundlagen bzw. Bestandteile wie insbesondere Hydroxyethylcellulose und/oder Wollwachsalkoholsalbe eingesetzt werden. Vorzugsweise wird eine hydrophile Salbe bzw. Creme, insbesondere eine wasserhaltige hydrophile Salbe bzw. Creme verwendet. Ferner werden bei dem erfindungsgemäßen Verfahren vorzugsweise Wirkstoffe, ausgewählt aus der Gruppe bestehend aus Vitamin, vorzugsweise Vitamin E, β-Carotin, Biotin und Co-Enzym Q10, sowie weitere wie vorstehend beschriebene Wirkstoffe zugegeben.

Ein weiterer Aspekt der vorliegenden Erfindung betrifft die Verwendung einer wie vorstehend beschriebenen erfindungsgemäßen pharmazeutischen Zubereitung, umfassend natürliche und/oder synthetische Wirkstoff(e) aus den Pflanzen Centella asiatica, Mahonia aquifolium, Viola tricolor, wobei ein Gehalt von 5 Gew.-% Zinkoxid und/oder ein Gehalt von 1 Gew.-% Perubalsam, bezogen auf die Gesamtzusammensetzung, ausgenommen ist, zur Herstellung eines Arzneimittels zur Behandlung von Neurodermitis, Psoriasis, Schuppenflechte, Windpocken, Varicellen, Herpes, Hautekzemen und/oder Hautjucken, insbesondere zur Behandlung von Neurodermitis.

Die nachfolgenden Beispiele dienen der Veranschaulichung der vorliegenden Erfindung.

### Beispiel 1: Rezeptur für eine pflanzliche Urtinktur zur äußeren Anwendung bei juckenden Ekzemen

| **Bestandteile** | **Gewichtsprozent** | **Monographien** |
|---|---|---|
| Acidum arsenicosum dil. D4 | 1 | Entzündungen in allen Geweben |
| Calendula officinalis | 2 | Schlecht heilende Wunden, Hauteiterungen |
| Centella asiatica | 5 | Hauterkrankungen mit Verdickungen und Juckreiz |
| Lytta vesicatoria (=Cantharis) dil. D4 | 1 | Akute Entzündungen der Haut mit Blasenbildung |
| Mahonia aquifolium | 1 | Trockene Hautausschläge |
| Perubalsam | 0 | |
| Semecarpus anacardium | 1 | Hautausschläge |
| Viola tricolor | 3 | Ekzeme |
| Zinkoxid | 0 | |

### Beispiel 2: Rezeptur für eine pflanzliche Urtinktur zur äußeren Anwendung bei juckenden Ekzemen

| **Bestandteile** | **Gewichtsprozent** | **Monographien** |
|---|---|---|
| Centella asiatica | 5 | Hauterkrankungen mit Verdickungen und Juckreiz |
| Mahonia aquifolium | 5 | Trockene Hautausschläge |
| Viola tricolor | 5 | Ekzeme |

Auch bei wiederholter Auftragung einer Salbe bzw. Creme basierend auf den vorstehend beschriebenen Rezepturen waren keine Gegenanzeigen oder Nebenwirkungen zu beobachten. Mit beiden Rezepturen blieb die herausragende juckreizlindernde Wirkung der pharmazeutischen Zubereitung gewährleistet.

Die Rezepturen gemäß den Beispielen 1 und 2 lassen sich zur Behandlung von allergischen Hauterkrankungen, atopisches Ekzem, Hauterkrankungen durch Pilze, Urtikaria, Neurodermitis, Psoriasis, Schuppenflechte, Hautekzemen, Hautjucken, entzündlichen Hauterkrankungen und/oder Viruserkrankungen, umfassend Windpocken, Varicellen, Herpes und Gürtelrose erfindungsgemäß verwenden.

## Patentansprüche

1. Verwendung einer pharmazeutischen Zubereitung zur Herstellung eines Arzneimittels zur Behandlung von allergischen Hauterkrankungen, atopisches Ekzem, Hauterkrankungen durch Pilze, Urtikaria, Neurodermitis, Psoriasis, Schuppenflechte, Hautekzemen, Hautjucken, entzündlichen Hauterkrankungen und/oder Viruserkrankungen, umfassend Windpocken, Varicellen, Herpes, Röteln und Gürtelrose, wobei die pharmazeutische Zubereitung natürliche und/oder synthetische Wirkstoff(e) als Extrakt(e), insbesondere als Urtinktur(en) aus den Pflanzen Centella asiatica, Mahonia aquifolium, Viola tricolor und/oder aus deren Pflanzenteilen umfasst, wobei bei einer Verwendung der pharmazeutischen Zubereitung zur Herstellung eines Arzneimittels zur Behandlung von allergischen Hauterkrankungen, atopisches Ekzem, Urtikaria, Neurodermitis, Psoriasis, Schuppenflechte, Hautekzemen, Hautjucken und/oder entzündlichen Hauterkrankungen die Zubereitung weniger als 1 Gew.-% Perubalsam und/oder weniger als 5 Gew.-% Zinkoxid, bezogen auf das Gesamtgewicht der Zubereitung, umfasst.

2. Verwendung der Zubereitung nach Anspruch 1,
**dadurch gekennzeichnet, dass** die Zubereitung ein alkoholischer Extrakt aus den Pflanzen Centella asiatica, Mahonia aquifolium, Viola tricolor und/oder aus deren Pflanzenteilen ist.

3. Verwendung der Zubereitung nach Anspruch 1 oder 2,
**dadurch gekennzeichnet, dass** die Zubereitung ein lipopholer Extrakt aus den Pflanzen Centella asiatica, Mahonia aquifolium, Viola tricolor und/oder aus deren Pflanzenteilen, vorzugsweise ein mittels verdichtetem Ethylen, Ethan oder Kohlendioxid unter überkritischen Bedingungen hergestellter Extrakt oder Alkylextrakt, insbesondere ein C₂ - C₈-Alkylextrakt, bevorzugt ein Kohlendioxid - Extrakt oder Hexan - Extrakt, ist.

4. Verwendung der Zubereitung nach einem der vorangehenden Ansprüche,
**dadurch gekennzeichnet, dass** die Zubereitung ≤ 0,5 Gew.-%, weiter bevorzugt ≤ 0,1 Gew.-% und besonders bevorzugt ≤ 0,01 Gew.-% Perubalsam, bezogen auf das Gesamtgewicht der Zubereitung, umfasst.

5. Verwendung der Zubereitung nach einem der vorangehenden Ansprüche,
**dadurch gekennzeichnet, dass** die Zubereitung ≤ 3 Gew.-%, weiter bevorzugt ≤ 1 Gew.-% und besonders bevorzugt ≤ 0,1 Gew.-% Zinkoxid, bezogen auf das Gesamtgewicht der Zubereitung, umfasst.

6. Verwendung der Zubereitung nach einem der vorangehenden Ansprüche,
**dadurch gekennzeichnet, dass** die Zubereitung kein Perubalsam und/oder Zinkoxid umfasst.

7. Verwendung der Zubereitung nach einem der vorangehenden Ansprüche,
**dadurch gekennzeichnet, dass** die Zubereitung eine Urtinktur mit Wirkstoff(en) aus den Pflanzen Centella asiatica, Mahonia aquifolium, Viola tricolor und/oder aus deren Pflanzenteilen umfasst.

8. Verwendung der Zubereitung nach einem der vorangehenden Ansprüche,
**dadurch gekennzeichnet, dass** die Zubereitung Wirkstoffe, vorzugsweise als Urtinktur, aus der Pflanze Centella asiatica und/oder aus deren Pflanzenteilen mit einem Wirkstoffgehalt von 1 Gew.-% bis 10 Gew.-%, vorzugsweise 3 Gew.-% bis 7 Gew.-%, besonders bevorzugt 4 Gew.-% bis 6 Gew.-% und am meisten bevorzugt 5 Gew.-%, bezogen auf das Gesamtgewicht der Zubereitung, umfasst.

9. Verwendung der Zubereitung nach einem der vorangehenden Ansprüche,
**dadurch gekennzeichnet, dass** die Zubereitung Wirkstoffe, vorzugsweise als Urtinktur, aus der Pflanze Mahonia aquifolium und/oder aus deren Pflanzenteilen mit einem Wirkstoffgehalt von 1 Gew.-% bis 10 Gew.-%, vorzugsweise 3 Gew.-% bis 7 Gew.-%, besonders bevorzugt 4 Gew.-% bis 6 Gew.-% und am meisten bevorzugt 5 Gew.-%, bezogen auf das Gesamtgewicht der Zubereitung, umfasst.

10. Verwendung der Zubereitung nach einem der vorangehenden Ansprüche,
**dadurch gekennzeichnet, dass** die Zubereitung Wirkstoffe, vorzugsweise als Urtinktur, aus der Pflanze Viola tricolor und/oder aus deren Pflanzenteilen mit einem Wirkstoffgehalt von 1 Gew.-% bis 10 Gew.-%, vorzugsweise 3 Gew.-% bis 7 Gew.-%, besonders bevorzugt 4 Gew.-% bis 6 Gew.-% und am meisten bevorzugt 5 Gew.-%, bezogen auf das Gesamtgewicht der Zubereitung, umfasst.

11. Verwendung der Zubereitung nach einem der vorangehenden Ansprüche,
**dadurch gekennzeichnet, dass** die Zubereitung ferner natürliche und/oder synthetische Farbstoffe, insbesondere ausgewählt aus der Gruppe, bestehend aus Curcumin, Riboflavin und β-Carotin, umfasst.

12. Verwendung der Zubereitung nach einem der vorangehenden Ansprüche,
**dadurch gekennzeichnet, dass** die Zubereitung als Salbe, Creme, Gel, Tablette oder Lösung, vorliegt, wobei eine hydrophile Salbe, insbesondere eine wasserhaltige hydrophile Salbe bevorzugt ist.

13. Verwendung der Zubereitung nach einem der vorangehenden Ansprüche,
**dadurch gekennzeichnet, dass** die Zubereitung als Salben- oder Cremegrundlage Hydroxyethylcellulose und/oder Wollwachsalkohol umfasst.

14. Verwendung der Zubereitung nach einem der vorangehenden Ansprüche,
**dadurch gekennzeichnet, dass** die Zubereitung ferner natürliche und/oder synthetische Wirkstoff(e) insbesondere in Form einer Urtinktur aus den Pflanzen Calendula officinalis und/oder Semecarpus anacardium und/oder aus deren Pflanzenteilen umfasst.

15. Verwendung der Zubereitung nach einem der vorangehenden Ansprüche,
**dadurch gekennzeichnet, dass** die Zubereitung ferner als Wirkstoff Acidum arsenicosum umfasst.

16. Verwendung der Zubereitung nach einem der vorangehenden Ansprüche,
**dadurch gekennzeichnet, dass** die Zubereitung ferner natürliche und/oder synthetische Wirkstoff(e) aus Lytta vesicatoria umfasst.

17. Verwendung der Zubereitung nach einem der vorangehenden Ansprüche,
**dadurch gekennzeichnet, dass** die Zubereitung ferner Wirkstoffe, ausgewählt aus der Gruppe, bestehend aus Vitamin, vorzugsweise Vitamin E, β-Carotin und Co-Enzym Q10, aufweist.

18. Verwendung der Zubereitung nach einem der vorangehenden Ansprüche,
**dadurch gekennzeichnet, dass** die Zubereitung ferner Wirkstoffe, ausgewählt aus der Gruppe, bestehend aus Curcumin und Curcuminwurzelstock-Extrakt umfasst.

19. Verwendung der Zubereitung nach einem der vorangehenden Ansprüche,
**dadurch gekennzeichnet, dass** die Zubereitung gebrauchsfertig ist.

20. Pharmazeutische Zubereitung,
**dadurch gekennzeichnet, dass** die pharmazeutische Zubereitung eine Zusammensetzung nach einem der vorangehenden Ansprüche 1 bis 17 aufweist, wobei ein Gehalt von 5 Gew.-% Zinkoxid und/oder ein Gehalt von 1 Gew.-% Perubalsam, bezogen auf die Gesamtzusammensetzung, ausgenommen ist.

21. Tube, enthaltend eine pharmazeutische Zusammensetzung nach einem der vorangehenden Ansprüche.

## Claims

1. Use of a pharmaceutical preparation for the manufacture of a medicament for the treatment of allergic skin diseases, atopic eczema, fungal skin diseases, urticaria, neurodermatitis, psoriasis, psora, skin eczemas, pruritus, inflammatory skin diseases and/or viral diseases, comprising chicken pox, varicella, herpes, rubella and shingles, whereas the pharmaceutical preparation comprises natural and/or synthetic active ingredient(s) in the from of an extract(s), in particular in the from of a mother tincture(s) from the plants centella asiatica, mahonia aquifolium, viola tricolor and/or from plant parts thereof, whereas in the use of the pharmaceutical preparation for the manufacture of a medicament for the treatment of allergic skin diseases, atopic eczema, urticaria, neurodermatitis, psoriasis, psora, skin eczemas, pruritus and/or inflammatory skin diseases the preparation comprises less than 1 percent by weight of peruvian balsam and/or less than 5 percent by weight of zinc oxide, based on the total weight of the preparation.

2. Use of the preparation according to claim 1, **characterized in that** the preparation is an alcoholic extract from the plants centella asiatica, mahonia aquifolium, viola tricolor and/or from plant parts thereof.

3. Use of the preparation according to claim 1 or 2, **characterized in that** the preparation is a lipopholic extract from the plants centella asiatica, mahonia aquifolium, viola tricolor and/or from plant parts thereof, preferably an extract or alkyl extract, in particular a C₂ - C₈-alkyl extract, preferably a carbon dioxide extract or a hexane extract, manufactured by means of compressed ethylene, ethane or carbon dioxide under supercritical conditions.

4. Use of the preparation according to any one of the preceding claims, **characterized in that** the preparation comprises ≤ 0.5 percent by weight, more preferred < 0.1 percent by weight and especially preferred ≤ 0.01 percent by weight of peruvian balsam, based on the total weight of the preparation.

5. Use of the preparation according to any one of the preceding claims, **characterized in that** the preparation comprises ≤ 3 percent by weight, more preferred ≤ 1 percent by weight and especially preferred ≤ 0.1 percent by weight of zinc oxide, based on the total weight of the preparation.

6. Use of the preparation according to any one of the preceding claims, **characterized in that** the preparation comprises no peruvian balsam and/or zinc oxide.

7. Use of the preparation according to any one of the preceding claims, **characterized in that** the preparation comprises a mother tincture with active ingredient(s) from the plants centella asiatica, mahonia aquifolium, viola tricolor and/or from plant parts thereof.

8. Use of the preparation according to any one of the preceding claims, **characterized in that** the preparation comprises active ingredients, preferably in the from of a mother tincture, from the plant centella asiatica and/or from plant parts thereof having an active ingredient content of 1 percent by weight to 10 percent by weight, preferably 3 percent by weight to 7 percent by weight, especially preferred 4 percent by weight to 6 percent by weight and most preferred 5 percent by weight, based on the total weight of the preparation.

9. Use of the preparation according to any one of the preceding claims, **characterized in that** the preparation comprises active ingredients, preferably in the from of a mother tincture, from the plant mahonia aquifolium and/or from plant parts thereof having an active ingredient content of 1 percent by weight to 10 percent by weight, preferably 3 percent by weight to 7 percent by weight, especially preferred 4 percent by weight to 6 percent by weight and most preferred 5 percent by weight, based on the total weight of the preparation.

10. Use of the preparation according to any one of the preceding claims, **characterized in that** the preparation comprises active ingredients, preferably in the from of a mother tincture, from the plant viola tricolor and/or from plant parts thereof having an active ingredient content of 1 percent by weight to 10 percent by weight, preferably 3 percent by weight to 7 percent by weight, especially preferred 4 percent by weight to 6 percent by weight and most preferred 5 percent by weight, based on the total weight of the preparation.

11. Use of the preparation according to any one of the preceding claims, **characterized in that** the preparation additionally comprises natural and/or synthetic coloring agents, in particular selected from the group consisting of curcumin, riboflavin and β-carotene.

12. Use of the preparation according to any one of the preceding claims, **characterized in that** the preparation is present in form of an ointment, cream, gel, tablet or solution, whereas a hydrophilic ointment, in particular a water-containing hydrophilic ointment, is preferred.

13. Use of the preparation according to any one of the preceding claims, **characterized in that** the preparation comprises hydroxyethyl cellulose and/or wool grease alcohol as ointment or cream base.

14. Use of the preparation according to any one of the preceding claims, **characterized in that** the preparation additionally comprises natural and/or synthetic active ingredient(s) in particular in the form of a mother tincture from the plants calendula officinalis and/or semecarpus anacardium and/or from plant parts thereof.

15. Use of the preparation according to any one of the preceding claims, **characterized in that** the preparation additionally comprises acidum arsenicosum as an active ingredient.

16. Use of the preparation according to any one of the preceding claims, **characterized in that** the preparation additionally comprises natural and/or synthetic active ingredient(s) from lytta vesicatoria.

17. Use of the preparation according to any one of the preceding claims, **characterized in that** the preparation additionally comprises active ingredients selected from the group consisting of vitamin, preferably vitamin E, β-carotene, and coenzyme Q10.

18. Use of the preparation according to any one of the preceding claims, **characterized in that** the preparation additionally comprises active ingredients selected from the group consisting of curcumin and curcumin rhizome extract.

19. Use of the preparation according to any one of the preceding claims, **characterized in that** the preparation is ready to use.

20. Pharmaceutical preparation, **characterized in that** the pharmaceutical preparation comprises a composition according to any one of the preceding claims 1 to 17, whereas a content of 5 percent by weight of zinc oxide and/or a content of 1 percent by weight of peruvian balsam, based on the total composition, is excluded.

21. Tube comprising a pharmaceutical composition according to any one of the preceding claims.

## Revendications

1. Utilisation d'une formulation pharmaceutique pour la préparation d'un médicament destiné au traitement d'allergies cutanées, de l'eczéma atopique, de maladies de la peau provoquées par des champignons, de l'urticaire, de la neurodermatite, du psoriasis, des affections cutanées de type eczémateux, de l'eczéma cutané, des démangeaisons cutanées, des maladies cutanées et/ou des maladies virales inflammatoires, y compris la varicelle, des éruptions varicelleuses, l'herpès, la rubéole et le zona, la formulation pharmaceutique comprenant un ou plusieurs principes actifs naturels et/ou synthétiques sous la forme d'extrait(s), en particulier sous la forme de teinture(s)-mère(s) issues des plantes Centella asiatica, Mahonia aquifolium, Viola tricolor et/ou de parties desdites plantes, dans laquelle, lors d'une utilisation de la formulation pharmaceutique pour la préparation d'un médicament destiné au traitement d'allergies cutanées, de l'eczéma atopique, de l'urticaire, de la neurodermatite, du psoriasis, des affections cutanées de type eczémateux, de l'eczéma cutané, des démangeaisons cutanées et/ou des maladies cutanées inflammatoires, la formulation comprend du baume du Pérou à concurrence de moins de 1 % en poids et/ou de l'oxyde de zinc à concurrence de moins de 5 % en poids, rapportés au poids total de la formulation.

2. Utilisation de la formulation selon la revendication 1, **caractérisée en ce que** la formulation est un extrait alcoolique issu des plantes Centella asiatica, Mahonia aquifolium, Viola tricolor et/ou de parties desdites plantes.

3. Utilisation de la formulation selon la revendication 1 ou 2, **caractérisée en ce que** la formulation est un extrait lipophile issu des plantes Centella asiatica, Mahonia aquifolium, Viola tricolor et/ou deux parties desdites plantes, de préférence un extrait préparé avec de l'éthylène, de l'éthane ou du dioxyde de carbone condensé dans des conditions supercritiques ou encore un extrait alkylique, en particulier un extrait alkylique en C₂-C₈, de préférence un extrait de dioxyde de carbone ou un extrait d'hexane.

4. Utilisation de la formulation selon l'une quelconque des revendications précédentes, **caractérisée en ce que** la formulation comprend du baume du Pérou à concurrence de ≤ 0,5 % en poids, de manière plus préférée à concurrence de ≤ 0,1 % en poids et de manière particulièrement préférée à concurrence de ≤ 0,01 % en poids, rapporté au poids total de la formulation.

5. Utilisation de la formulation selon l'une quelconque des revendications précédentes, **caractérisée en ce que** la formulation comprend de l'oxyde de zinc à concurrence de ≤ 3 % en poids, de manière plus préférée à concurrence de ≤ 1 % en poids et de manière particulièrement préférée à concurrence de ≤ 0,1 % en poids.

6. Utilisation de la formulation selon l'une quelconque des revendications précédentes, **caractérisée en ce que** la formulation ne comprend pas de baume du Pérou et/ou de l'oxyde de zinc.

7. Utilisation de la formulation selon l'une quelconque des revendications précédentes, **caractérisée en ce que** la formulation comprend une teinture-mère contenant un ou plusieurs principes actifs choisis parmi le groupe de plantes comprenant Centella asiatica, Mahonia aquifolium, Viola tricolor et/ou des parties desdites plantes.

8. Utilisation de la formulation selon l'une quelconque des revendications précédentes, **caractérisée en ce que** la formulation comprend des principes actifs, de préférence sous la forme d'une teinture-mère issue de la plante Centella asiatica et/ou de parties de ladite plante, avec une teneur en substances actives de 1 % en poids à 10 % en poids, de préférence de 3 % en poids à 7 % en poids, de manière particulièrement préférée de 4 % en poids à 6 % en poids et de manière de loin préférée à concurrence de 5 % en poids, rapportés au poids total de la formulation.

9. Utilisation de la formulation selon l'une quelconque des revendications précédentes, **caractérisée en ce que** la formulation comprend des principes actifs, de préférence sous la forme d'une teinture-mère issue de la plante Mahonia aquifolium et/ou de parties de ladite plante, avec une teneur en substances actives de 1 % en poids à 10 % en poids, de préférence de 3 % en poids à 7 % en poids, de manière particulièrement préférée de 4 % en poids à 6 % en poids et de manière de loin préférée à concurrence de 5 % en poids, rapportés au poids total de la formulation.

10. Utilisation de la formulation selon l'une quelconque des revendications précédentes, **caractérisée en ce que** la formulation comprend des principes actifs, de préférence sous la forme d'une teinture-mère issue de la plante Viola tricolor et/ou de parties de ladite plante, avec une teneur en substances actives de 1 % en poids à 10 % en poids, de préférence de 3 % en poids à 7 % en poids, de manière particulièrement préférée de 4 % en poids à 6 % en poids et de manière de loin préférée à concurrence de 5 % en poids, rapportés au poids total de la formulation.

11. Utilisation de la formulation selon l'une quelconque des revendications précédentes, **caractérisée en ce que** la formulation comprend en outre des colorants naturels et/ou synthétiques, en particulier choisis parmi le groupe constitué par la curcumine, la riboflavine et la β-carotine.

12. Utilisation de la formulation selon l'une quelconque des revendications précédentes, **caractérisée en ce que** la formulation est présente sous la forme d'un onguent, d'une crème, d'un gel, d'un comprimé ou d'une solution, un onguent hydrophile, un onguent hydrophile aqueux étant particulièrement préféré.

13. Utilisation de la formulation selon l'une quelconque des revendications précédentes, **caractérisée en ce que** la formulation comprend, à titre de substrat de l'onguent ou de la crème, de l'hydroxyéthylcellulose et/ou de la lanoline.

14. Utilisation de la formulation selon l'une quelconque des revendications précédentes, **caractérisée en ce que** la formulation comprend en outre un ou plusieurs principes actifs naturels et/ou synthétiques en particulier sous la forme d'une teinture-mère issue des plantes Calendula officinalis et/ou Semecarpus anacardium et/ou de parties desdites plantes.

15. Utilisation de la formulation selon l'une quelconque des revendications précédentes, **caractérisée en ce que** la formulation comprend en outre, à titre de principe actif, Acidum arsenicosum.

16. Utilisation de la formulation selon l'une quelconque des revendications précédentes, **caractérisée en ce que** la formulation comprend en outre un ou plusieurs principes actifs naturels et/ou synthétiques issus de Lytta vesicatoria.

17. Utilisation de la formulation selon l'une quelconque des revendications précédentes, **caractérisée en ce que** la formulation comprend en outre des principes actifs choisis parmi le groupe constitué par des vitamines, de préférence la vitamine E, la β-carotine et la coenzyme Q10.

18. Utilisation de la formulation selon l'une quelconque des revendications précédentes, **caractérisée en ce que** la formulation comprend en outre des principes actifs choisis parmi le groupe constitué par de la curcumine et de l'extrait de la racine de curcuma.

19. Utilisation de la formulation selon l'une quelconque des revendications précédentes, **caractérisée en ce que** la formulation est prête à l'emploi.

20. Formulation pharmaceutique **caractérisée en ce que** la formulation pharmaceutique présente une composition selon l'une quelconque des revendications 1 à 17, une teneur d'oxyde de zinc à concurrence de 5 % en poids et/ou une teneur de baume du Pérou à concurrence de 1 % en poids, rapportés à la composition totale, étant exclues.

21. Tube contenant une composition pharmaceutique selon l'une quelconque des revendications précédentes.
